# EUROPEAN PATENT SPECIFICATION

(11) **EP 4 483 776 B1**
(45) Date of publication and mention of the grant of the patent: **05.08.2026**
(21) Application number: 23182208.1
(22) Date of filing: 28.06.2023
(51) Int. Cl.: A61B 1/00, A61B 1/012, A61B 1/015, A61B 1/018, A61B 1/005, A61B 1/04

(54) **ENDOSCOPE COMPRISING A Y-CONNECTOR WITH OFFSET SUCTION AND INSTRUMENT INSERTION CHANNELS**
ENDOSKOP MIT Y-VERBINDER MIT VERSETZTEN ANSAUG- UND INSTRUMENTENEINFÜHRKANÄLEN
ENDOSCOPE COMPRENANT UN CONNECTEUR EN Y AVEC CANAUX D'ASPIRATION ET D'INSERTION D'INSTRUMENT DÉCALÉS

(43) Date of publication of application: 01.01.2025
(73) Proprietor: Ambu A/S, 2750 Ballerup (DK)
(72) Inventor: MORTON, Alistair David, 2300 København (DK)
(74) Representative: Winter, Brandl - Partnerschaft mbB

(56) References cited:
- DE-A1- 102020 133 405
- JP-A- 2002 219 096
- JP-A- 2011 177 355
- US-A1- 2023 157 527

## Description

The present disclosure relates to an endoscope, in particular a single-use endoscope, comprising: an endoscope handle or interface; and an insertion cord extending from the endoscope handle or interface and configured to be inserted into a patient's body cavity; the endoscope handle or interface comprising a Y-connector having: a first branch or socket having a first opening from which a first channel extends; a second branch or socket having a second opening from which a second channel extends; and a third branch or socket having a third opening, wherein the first channel and the second channel join each other in a third joined channel and the third joined channel extends inside the third branch or socket towards the third opening; wherein the first channel and the third joined channel form together an instrument insertion channel of the Y-connector, the second channel and the third joined channel form together a suction channel of the Y-connector, and a working channel tube is directly or indirectly connected to the third joined channel and extends from the endoscope handle or interface into the insertion cord. The present disclosure also relates to a visualization system comprising such an endoscope and a monitor connectable to the endoscope.

### Related art

Endoscopes and similar specialized instruments such as bronchoscopes, arthroscopes, colonoscopes, laparoscopes, gastroscopes, and duodenoscopes are well-known from the related art and are used for visual examination and diagnosis of hollow organs and body cavities, as well as to assist in surgery, e.g. for a targeted tissue sampling. Both reusable and disposable endoscopes are known from the related art. Known endoscopes usually comprise: an endoscope handle or interface with a working channel access port; an insertion cord extending from the endoscope handle or interface to be inserted into a patient's body cavity and comprising an insertion tube, a bending section, and a distal tip unit; and a working channel. The working channel extends from the working channel access port at the endoscope handle to the distal tip unit.

The working channel is usually formed by at least: a Y-connector or biopsy connector; a (flexible) working channel tube arranged inside the endoscope handle, the insertion tube and the bending section; and a distal tip housing. A surgical instrument or tool may be guided through the working channel into the patient's body cavity. In particular, the surgical instrument or tool may be inserted into the working channel access port and may be guided through the Y-connector, the working channel tube and the distal tip housing into the patient's body cavity via an opening provided in the distal tip unit. In addition to allowing insertion of surgical instruments or tools - like a forceps for taking tissue samples - into the patient's body cavity, the working channel also has the purpose of enabling suction of fluids from the patient's body cavity. The Y-connector is thus in other words a branched tubing component arranged in the proximal endoscope handle or interface of the endoscope, wherein the Y-connector/ the branched tubing component joins or connects a suction tube/ a suction channel and a tool/ instrument insertion channel with a working channel tube to allow insertion of surgical tools or instruments into the working channel tube and to allow suction of fluids out of the working channel tube.

Y-shaped biopsy connectors or Y-connectors are well known from the related art. Known Y-connectors often comprise instrument insertion channels consisting of two merging straight channels that are angled with respect to each other. An example of a Y-connector with two straight channels is e.g. disclosed in WO 2007/117750 A2.

When a surgical instrument is inserted into an instrument insertion channel consisting of two merging straight channels angled with respect to each other, bending of the surgical instrument requires a certain bending force. Because of the bending force, the user inserting the surgical instrument into the working channel tube feels resistance while inserting the instrument. This resistance in the insertion process is disadvantageous as it compromises a user experience during insertion of the surgical instrument. In particular, the user receives unwanted, disadvantageous haptic feedback when the surgical instrument is bent inside the Y-connector.

Single-use or disposable endoscopes are usually low-cost and lightweight endoscopes which are only used on one single patient and which are disposed after one single use. Single-use endoscopes are thus usually optimized for one single use. They have basically the advantage that cross-contamination between patients is prevented, but also that single-use endoscopes optimize workflow and reduce cost while saving patient's lives and improving patient care. They optimize workflow and reduce cost because they are always ready when needed without the traditional large-scale capital and repair budgets required for reusable endoscopes. For single-use endoscopes, it is desirable to reduce manufacturing and assembly costs. Single-use endoscope usually have a limited number of elements, which are usually manufactured with a low-cost material (polymer/ plastic/ resin) in a low-cost manufacturing process (plastic/ injection molding) and which can be easily assembled.

The related art include some examples of single-use endoscopes having a Y-connector manufactured in a plastic material by injection molding, but there is still room for improvement.

WO 2022/128689 A1 e.g. discloses a single-use endoscope comprising a Y-connector, wherein the Y-connector is a multi-piece connector comprising a first part and a separate second part that are attached to each other by gluing The second part is a lid or cover. Both parts are made in a plastic material by injection molding.

EP 4 183 310 A1 discloses another single-use endoscope comprising a Y-connector. The instrument insertion channel of the Y-connector has a curved shape in the shape of a banana in order to reduce a resistance, which the user feels when inserting a surgical tool or instrument into the working channel. The Y-connector disclosed in EP 4 183 310 A1 is also molded in two pieces - a first main part and a second lid or cover part - which are glued together.

Even with the Y-connector disclosed in EP 4 183 310 A1 there may be a certain unpleasant resistance when a surgical tool or instrument is inserted into the instrument insertion channel of the Y-connector, in particular when the surgical tool or instrument reaches a joined channel portion of the instrument insertion channel, which joined channel portion is a channel portion of the Y-connector serving both for inserting the tool or instrument into the working channel tube and for sucking fluids from the working channel tube. Any sudden resistance could be interpreted by the user that there is something wrong, e.g. that the endoscope is faulty or the instrument is hitting a body lumen wall of the patient, which could be detrimental. Moreover, the Y-connectors disclosed in WO 2022/128689 A1 and in EP 4 183 310 A1 have manufacturing and assembly drawbacks, in particular since two parts are manufactured separately and assembled afterwards, which adds to the complexity and cost of manufacture.

DE 10 2020 133 405 A1, US 2023 / 157 527 A1 and JP 2001 177 355 A all disclose endoscopes with a Y-connector having an instrument insertion channel and a suction channel. The respective suction channels are straight and have a straight suction channel axis.

### Brief description of the disclosure

In view of the above-described problems, it is an object of the present disclosure to avoid or at least to mitigate the disadvantages of the related art, in particular to provide an endoscope, preferably single-use endoscope, having a Y-connector which provides or enables simple and economic manufacturing and/or assembly and in which a surgical tool or instrument can be inserted into a working channel with low resistance.

This object is solved by an endoscope in accordance with claim 1 and by a visualization system in accordance with claim 14. Advantageous aspects of the present disclosure are claimed in the dependent claims and/or are described herein below.

The present disclosure relates to an endoscope comprising: an endoscope handle or interface; and an insertion cord extending from the endoscope handle or interface and configured to be inserted into a patient's body cavity. The endoscope handle or interface comprises a Y-connector (biopsy connector) having: a first branch or socket having a first opening from which a first channel extends; a second branch or socket having a second opening from which a second channel extends; and a third branch or socket having a third opening, wherein the first channel and the second channel join each other in a third joined channel and the third joined channel extends inside the third branch or socket towards the third opening. The first channel and a first portion of the third joined channel form together an instrument insertion channel of the Y-connector and the second channel and a second portion of the third joined channel form together a suction channel of the Y-connector. A working channel tube is directly or indirectly connected to the third joined channel and extends from the endoscope handle or interface into the insertion cord. The instrument insertion channel and the suction channel are at least in portions displaced or offset with respect to each other.

The instrument insertion channel is a curved channel and has a curved instrument insertion channel central axis or line, wherein the curved instrument insertion channel central axis or line defines a first plane, wherein the curved instrument insertion channel central axis or line lies completely in the first plane, and wherein the suction channel central axis or line does not lie in the first plane.

According to the present disclosure, an offset or a displacement of the instrument insertion channel with respect to the suction channel preferably means that (a central axis or line of) the suction channel is offset or displaced from a plane defined by (a central axis or line of) the (curved) instrument insertion channel. The suction channel may e.g. be parallel to said plane or may intersect said plane. Alternatively one could also say that the instrument insertion channel and the suction channel are preferably decoupled from each other due to their offset/ displaced arrangement.

In related art endoscopes, the suction channel basically lies in the plane defined by the instrument insertion channel. The second branch or socket usually has a branch opening inside the Y-connector, wherein the second channel extends between the branch opening and the second opening. It has been found that when a surgical tool or instrument is guided or inserted into the instrument insertion channel of related art endoscopes, the surgical tool or instrument is not suitably guided in an area of said branch opening. In particular, a tip of the surgical tool or instrument may lose its contact with a wall surface of the instrument insertion channel, which may lead to the surgical tool or instrument bumping into a wall surface of the third joined channel resulting in an unpleasant resistance experienced by the user of the endoscope.

According to the present disclosure, due to the displacement/ the offset of the instrument insertion channel with respect to the suction channel, there is advantageously a continuous guidance of the surgical instrument or tool by wall surfaces of the instrument insertion channel, i.e. the surgical tool or instrument preferably does not lose its contact with a wall surface of the instrument insertion channel on its way through the instrument insertion channel of the Y-connector.

Moreover, due to the displacement of the instrument insertion channel with respect to the suction channel, the instrument insertion channel must not be excessively curved in a transition area between the first channel and the third joined channel. There is thus preferably provided a smoothly curved instrument insertion channel according to the present disclosure. The instrument insertion channel is preferably (smoothly) curved over its entire extension between the first opening and the third opening, i.e. there are preferably no straight portions or only a short straight portion near the third opening. E.g. the radius of curvature may be the same over the extension of the instrument insertion channel or may vary only by up to 30%, preferably only by up to 20%, especially preferred only by up to 10%. According to the present disclosure, the smooth curvature of the instrument insertion channel is enabled due to the offset/ displaced arrangement of the instrument insertion channel with respect to the suction channel. The smooth curvature of the instrument insertion channel makes it possible that the Y-connector can advantageously be manufactured as a single piece, i.e. as a unitary component, by plastic injection molding. In particular, core parts can be removed after the injection molding process more easily due to the smooth curvature. The offset channels may allow a functional design of the (molding/casting) cores. By offsetting the two channels, the cores can be removed easily after the molding process and the Y-connector can be manufactured in one single manufacturing step. Thus, no assembly of different components or parts of the Y-connector is necessary. This allows for a low-cost and quick production of the Y-connector and results in low component costs for the production of the single-use endoscope. Especially, there is no need for a skilled worker to glue the small parts together. Furthermore, no further inspection of the assembled Y-connector is necessary. The displacement or offset according to the present disclosure therefore makes it possible to simply and economically manufacture the Y-connector as a single/ integral component and also to insert a surgical tool or instrument into the working channel tube via the Y-connector with low resistance.

In particular, the offset or displacement of the instrument insertion channel with respect to the suction channel is reached by dividing the third joined channel at least in portions, preferably entirely, into a first portion and a second portion. By providing a first portion of the third joined channel which is continuous with the first channel, or in other words which is the extension of the first channel, it is advantageously reached that the surgical tool or instrument is suitably guided through the instrument insertion channel without losing contact to a wall surface of the instrument insertion channel on its way through the same. By providing a second portion of the third joined channel which is continuous with the second channel, or in other words which is the extension of the second channel, it is advantageously reached that a dedicated and continuous suction channel is provided, enabling fluid or mucus being sucked through the same.

The first portion and the second portion are both portions of the third joined channel, i.e. they are connected and together form the third joined channel. The first portion and the second portion preferably are arranged laterally next to each other. By providing the connected first and second portions, it is advantageously reached that the instrument insertion channel and the suction channel do not use the same lumen having an essentially round or circular cross-section inside the third branch or socket, but use lumens/ channel portions adjacent to each other inside the third branch or socket. Therefore, advantageously both the suction channel and the instrument insertion channel have their respective own lumens inside the third branch or socket, with the two lumens being connected and forming together the third joined channel. With the suction channel and the instrument insertion channel being decoupled from each other at least in portions, negative interference effects between the two channels/ lumens can be reduced, e.g. pressure loss. For example, an impact of the fluid flow in the suction channel on the instrument insertion through the instrument insertion channel can be reduced. Furthermore, a negative impact on the suction performance due to an instrument being arranged in the third joined channel can also be reduced.

When a (surgical) tool or instrument is inserted into the Y-connector, the tool or instrument enters through the first opening of the first branch or socket and is guided through the first channel and the first portion of the third joined channel into the working channel tube directly or indirectly connected to the third joined channel. The (surgical) tool or instrument is smoothly bent when guided through the instrument insertion channel. When fluid or mucus is sucked or pumped from the working channel tube through the Y-connector, fluid or mucus enters through the third opening of the third branch or socket and is sucked through the second portion of the third joined channel and the second channel into a suction tube connected to the second branch or socket. The second branch or socket may be connected to a suction valve provided in the endoscope handle via the suction tube, and - via the suction valve - to an external suction device like a vacuum pump.

The instrument insertion channel being offset/ displaced with respect to the suction channel at least in portions means that it is basically conceivable according to the present disclosure that there is provided a portion in the third joined channel in which no offset/ displacement is provided between the channels, i.e. in which one common channel having a round/ circular cross-section is provided. However, it is advantageous according to the present disclosure that the instrument insertion channel and the suction channel are entirely displaced or offset with respect to each other, i.e. a central axis or line of the instrument insertion channel does not correspond to a central axis or line of the suction channel inside the Y-connector, not even in portions. "Displaced or offset with respect to each other" may additionally or alternatively also be understood such that both channels are distanced from each other in at least a section or portion of the respective channels. Central axes or lines, which extend along the respective channels, are preferably not identical and are therefore not super-imposed. The portions in which the suction channel and the instrument insertion channel are displaced or offset with respect to each other may extend over the entire channels, i.e. over the complete length of the respective channels. The offset portions may also extend only over a part or portion of the respective channels. Thus, the suction channel and the instrument insertion channel may alternatively overlap or intersect in a portion of the respective channels.

The first channel and the second channel preferably join each other, i.e. merge into each other, to form the third joined channel. The three channels preferably form together a Y-shape/ are preferably arranged in a Y-shape at least in a side view.

The endoscope according to the present disclosure is preferably a low-cost, lightweight, single-use endoscope, which is intended to be disposed after use. This means that the endoscope is preferably optimized for one single use. The endoscope preferably has a limited number of elements, which are preferably manufactured with a low-cost material (polymer/ plastic/ resin) in preferably a low-cost manufacturing process (plastic/ injection molding) and which can be easily assembled. The endoscopes according to the present disclosure are primarily made of polymer materials, which further reduce the environmental impact. Compared to traditional reusable endoscopes, the focus of the present disclosure is to provide an endoscope that is only used once and which thus does not have to withstand rather aggressive cleaning or sterilization processes and general harsh handling over the life cycle of the endoscope.

The Y-connector is preferably attached to a handle housing of the endoscope handle or interface, i.e. is preferably integrated into the endoscope handle or interface. A working channel access port or biopsy port is preferably provided on an outer side of the handle housing. The working channel access port is preferably connected to the first branch or socket so that a surgical instrument or tool can be inserted into the working channel via the working channel access port.

Preferably, the suction channel may be a straight channel and may have a suction channel central axis being a central axis of the suction channel. A proximal working channel tube portion of the working channel tube may be directly or indirectly connected to the third joined channel. The proximal working channel tube portion may comprise a proximal working channel tube portion central axis being a central axis of the proximal working channel tube portion, and the suction channel central axis may extend parallel to the proximal working channel tube portion central axis. The suction channel may form a continuation of the proximal working channel tube portion. The suction channel may be connected to the proximal working channel tube portion without any steps or barriers. The straight suction channel may reduce the flow resistance of the liquids/ mucus sucked through the suction channel of the Y-connector, i.e. reduce the pressure loss, and thereby increase the performance of the endoscope. Especially the connection between the proximal working channel tube portion and the suction channel may provide a smooth transition preferably without any steps affecting the fluid flow.

The proximal working channel tube portion may be a section or portion of the working channel tube. The proximal working channel tube portion is preferably the portion at the proximal end of the working channel tube and may be connected to the Y-connector, preferably to the third branch or socket of the Y-connector.

Preferably, the suction channel central axis may correspond to the proximal working channel tube portion central axis. Thus, the suction channel and the proximal working channel tube portion may have the same axis. As a result, the liquid flow from the working channel tube into the suction channel advantageously experiences less resistance. Therefore, the suction power or pump pressure to remove liquid from the patient's body cavity may be further decreased.

According to a preferred aspect of the present disclosure, a cross-sectional area of the suction channel may increase in the second channel towards the second opening. More concrete, a diameter or circumference of the suction channel may increase in the second channel towards the second opening, i.e. in proximal direction. With the increasing cross-sectional area of the suction channel, the fluid flow through the suction channel may be facilitated. Furthermore, it may be easier to remove the molding core forming the second channel after the manufacturing of the Y-connector, preferably after the injection molding of the Y-connector.

The curved instrument insertion channel may be described as having a curved elongated cylindrical shape and may have a preferably circular cross-section. The instrument insertion channel central axis may be defined as an axis (of the cylinder) extending along the instrument insertion channel. The instrument insertion channel central axis preferably follows any curves of the instrument insertion channel so that a distance is kept constant between the instrument insertion channel central axis and a wall surface of the curved instrument insertion channel. The instrument insertion channel central axis may also be defined as a connection of center points of the respective (circular) cross-sections of the instrument insertion channel.

The first plane is defined by the curved instrument insertion channel central axis such that the curved instrument insertion channel central axis lies completely in the first plane. Said differently, the curved instrument insertion channel central axis may be part of the first plane. The suction channel central axis may not lie in the first plane. That means, the suction channel central axis may be angled with respect to the first plane or may extend parallel to the first plane.

The first channel may be formed as a curved channel having a curvature at least in sections inside the first branch or socket and/or the first portion of the third joined channel may be formed as a curved channel having a curvature at least in sections inside the third branch or socket. It is to be understood that "having a curvature at least in sections" means that either the whole channel inside the respective branch or socket may be curved, or only a section/ portion of the channel may be curved. The curvature of the first and/or third channel may result e.g. in a banana-shape of the instrument insertion channel formed by the first channel and the third joined channel.

The curvature of the first channel and/or of (the first portion of) the third joined channel may decrease the force needed to bend the instrument and therefore provides less resistance when the instrument is inserted into the Y-connector and guided through the instrument insertion channel. When the first channel is curved, a contacting point of the inserted instrument on the inner wall surface of the instrument insertion channel may be in the first channel and the instrument may be smoothly guided along the first channel and the first portion of the third joined channel and may be smoothly bent over a long distance. Therefore, resistance when inserting the instrument into the Y-connector may be appropriately reduced.

The first channel may be considered to be curved away from the second channel. In a longitudinal sectional view, the first channel may have a curvature at least in sections. The curvature may be described to have a radial inner side/ wall surface and a radial outer side/ wall surface. The radial inner side (inside of the curve) may be considered as the side of the first channel in which a course when running around the curved portion/ section is smaller compared to the radial outer side (outside of the curve). The first channel may be curved in a way that the radially outer side is closer to the second channel than the radially inner side.

The curved instrument insertion channel may allow the instrument to be inserted from the biopsy port to the working channel tube preferably without any obstacles. The smooth transition between the first channel and the first portion of the third joined channel may let the instrument pass through the channels with low resistance.

According to a preferred aspect of the present disclosure, the suction channel central axis may be parallel to the first plane or may cross the first plane at an intersection point. The suction channel central axis advantageously does not lie in the first plane, i.e. is not a part of the first plane. The suction channel central axis not lying in the first plane means that the suction channel and the instrument insertion channel are displaced or offset at least in portions.

In related art Y-connectors, the axes of the suction channel and the instrument insertion channel lie in the same plane. The channels may merge into each other in the third joined channel and provide a common channel in which the suction channel and the instrument insertion channel overlap and form one common channel having an essentially round/ circular cross-section. The offset channels according to the present disclosure may decouple the respective channels from each other.

Preferably, the instrument insertion channel and the suction channel may merge inside the third branch or socket and may form a cross-section in the shape of connected or intersecting circles inside the third branch or socket. Thus, the instrument insertion channel and the suction channel preferably run together inside the third branch or socket. The merging and adjacent channels may be positioned with respect to each other such that the circular cross-sections of both channels intersect inside the third branch or socket. Thus, the cross-section of the third branch or socket may have a shape of the connected circles of the merging channels. As a result, two connected but decoupled channels/ lumens are suitably provided.

The cross-sections of the instrument insertion channel and of the suction channel are preferably not arranged as concentric circles with respect to each other in the third branch or socket. Said differently, the instrument insertion channel central axis and the suction channel central axis do not correspond to each other. Therefore, the instrument insertion channel and the suction channel may be arranged adjacent or next to each other (in the third branch or socket). The adjacent channels may decouple the instrument insertion function and the suction function of the respective channels. Furthermore, the molding cores may be removed more easily from the adjacent channels.

Preferably, a cross-section of the first channel and/or a cross-section of the second channel is at least in sections essentially circular, and a cross-section of the third joined channel is preferably not circular.

According to a preferred aspect of the present disclosure, the third opening may have the shape of connected or intersecting circles. I.e. preferably, there is provided an offset or displacement of the instrument insertion channel with respect to the suction channel over their entire lengths, i.e. not only in portions.

The offset/ displacement of the suction channel with respect to the instrument insertion channel may be such that the suction channel is parallel to the instrument insertion channel. In this case the suction channel central axis would not intersect the first plane defined by the instrument insertion channel central axis or line.

Preferably, a cross-section, in particular a diameter or circumference, of the suction channel may be smaller than a cross-section, in particular a diameter or circumference, of the instrument insertion channel. The instrument insertion channel may thus have a bigger dimension than the suction channel. A bigger instrument insertion channel allows a smooth insertion of a surgical tool or instrument into the instrument insertion channel. For a smooth flow of the liquids or mucus sucked through the suction channel, a smaller cross-section is basically sufficient. The cross-section of the instrument insertion channel is preferably chosen such that typical surgical tools or instruments which are to be inserted into the working channel via the instrument insertion channel, can be inserted through the same.

Preferably, the Y-connector is manufactured as an integral piece, preferably from a plastic material, especially preferred by injection molding. Therefore, it may be necessary to remove the cores used in the injection molding process after injection molding in order to receive the finished Y-connector. A rather long molding core may be provided inside the first branch or socket, which forms the first channel. A shorter molding core may be provided inside the second branch or socket, which forms the second channel. The offset suction and instrument insertion channels and the smooth curvature of the instrument insertion channel may allow the molding cores to be removed easily. Thus, it may not be necessary to assemble the Y-connector from multiple parts or components. Alternatively, the Y-connector may also be manufactured by another manufacturing method, e.g. 3D printing.

Preferably, the first channel of the Y-connector is formed to be configured to pre-bend the instrument inserted into the instrument insertion channel inside the first branch or socket through its curvature, in particular through three-point bending. As discussed above, the first channel may be curved at least in sections. The curve of the first channel may have the radially inner side or wall surface and the radially outer side or wall surface. The inner side of the curve may be defined as the side of the curve with a smaller distance to run around the curve. The instrument inserted into the instrument insertion channel may be pre-bent in the first channel.

The object of the present disclosure is also solved by a visualization system comprising an endoscope as described above and a monitor connectable to the endoscope.

### Brief description of the figures

The disclosure is explained in more detail below using preferred embodiments and referring to the accompanying figures.
- Fig. 1: shows a side view of an endoscope according to the present disclosure.
- Fig. 2: schematically shows a Y-connector with its internal channels and with a working channel tube connected to the same.
- Fig. 3: shows a perspective view of a Y-connector according to a preferred embodiment of the present disclosure.
- Fig. 4: shows another perspective view of the Y-connector according to the preferred embodiment of the present disclosure.
- Fig. 5: shows another perspective view of the Y-connector according to the preferred embodiment of the present disclosure.
- Fig. 6: shows a perspective view of a Y-connector being inserted into a portion of an endoscope handle or interface of the endoscope according to the preferred embodiment of the present disclosure.

The figures are schematic in nature and serve only to understand the disclosure. The features of the different embodiments can be interchanged among each other.

### Detailed description of the figures

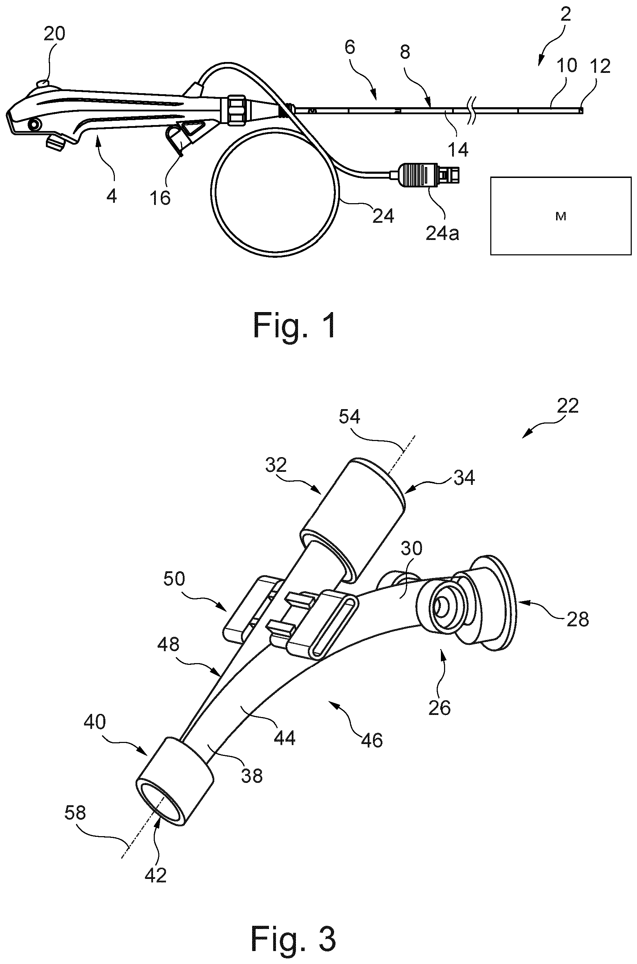

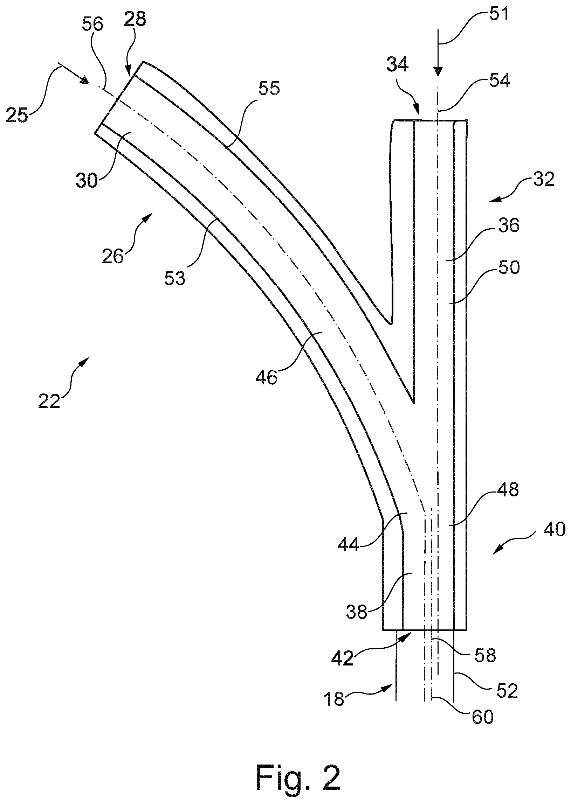

Fig. 1 shows a side view of an endoscope 2 according to the present disclosure, which is preferably a single-use endoscope. The endoscope 2 comprises: a proximal endoscope handle or interface 4 and an insertion cord 6 extending distally from the endoscope handle 4. The insertion cord 6 is configured to be inserted into a patient's body cavity and comprises an insertion tube 8, a bending section 10 and a distal tip unit 12. The endoscope 2 further comprises a working channel 14 which extends from a working channel access port or biopsy port 16 provided at the endoscope handle 4 to the distal tip unit 12. The working channel 14 comprises a working channel tube 18 (shown in Fig. 2), which is arranged inside the endoscope handle 4, the insertion tube 8, and the bending section 10. The endoscope handle 4 comprises an operating unit 20, formed as a lever, for bending the bending section 10 of the insertion cord 6. The working channel tube 18 and the working channel access port 16 are connected via a Y-connector 22. The endoscope 2 is connectable to a monitor M (schematically illustrated) via a connecting cable 24 comprising an electrical connector 24a insertable in a socket (not shown) of the monitor M. Alternatively the endoscope 2 may be wirelessly connectable to the monitor M, e.g. by a wireless HDMI connection.

When a surgical instrument or tool 25 is inserted into the working channel 14, the surgical instrument or tool 25 is inserted into the working channel access port 16 and guided through the Y-connector 22 into the working channel tube 18.

Fig. 2 schematically shows the interior of the Y-connector 22. The Y-connector 22 comprises a first branch or socket 26 having a first opening 28. A first channel 30, preferably a biopsy channel, extends from the first opening 28 inside the first branch or socket 26. The Y-connector 22 further comprises a second branch or socket 32 with a second opening 34. A second channel 36 extends from the second opening 34. The first channel 30 and the second channel 36 join each other/ merge into each other to form a third joined channel 38. The third joined channel 38 extends inside a third branch or socket 40 towards a third opening 42. The first channel 30 and a first portion 44 of the third joined channel 38 form together an instrument insertion channel 46, through which the surgical instrument or tool 25 is inserted into the working channel tube 18. The second channel 36 and a second portion 48 of the third joined channel 38 form together a suction channel 50, through which fluid and potential smoke and fume is sucked out of the patient's body cavity. The first opening 28 or the first branch or socket 26 is connected to the working channel access port/ biopsy port 16, which is configured to initially receive the surgical instrument or tool 25. The working channel access port 16 is an elastic part having an adapter portion, e.g. for luer-lock. The second opening 34 is connected to a suction tube 51. The third opening 42 is connected, preferably directly, to a proximal working channel tube portion 52 of the working channel tube 18. The third opening 42 may be connected to the working channel tube via a rotating joint.

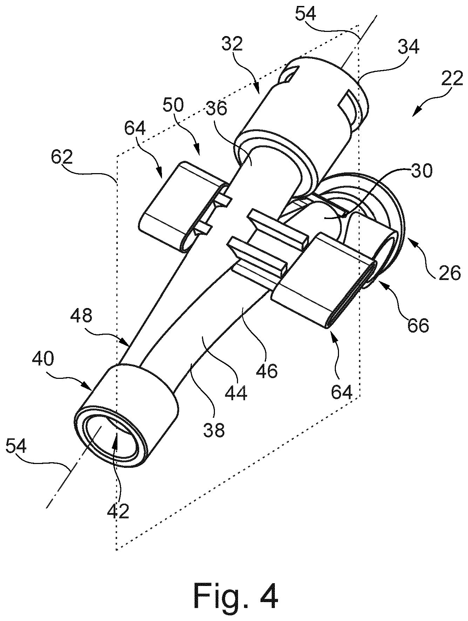

With reference also to Fig. 3 and Fig. 4, the first channel 30 may be described to be curved away from the second channel 36. In the longitudinal sectional view of Fig. 2, a curved portion of the first channel 30 comprises a curvature and a curve of the first channel 30 has two sides, a radial inner side/ wall surface 53 and a radial outer side/ wall surface 55. The radially outer wall surface 55 is closer to the second channel 36 than the radially inner wall surface 53. The third joined channel 38 comprises the first portion 44 connected to and forming a direct extension of the first channel 30 and the second portion 48 connected to and forming a direct extension of the second channel 36. The first portion 44 has a curvature. The curvature of the first portion 44 of the third joined channel 38 is curved to form an essentially continuous curvature with the curved portion of the first channel 30. The curved first portion 44 comprises a radially inner wall surface and a radially outer wall surface. In particular, there is provided both a direct transition without kink between the radially inner wall surface of the first channel 30 and the radially inner wall surface of the first portion 44 of the third joined channel 38 and a direct transition without kink between the radial outer wall surface of the first channel 30 and the radial outer wall surface of the first portion 44 of the third joined channel 38. Said differently, the first portion 44 preferably continues a trajectory of the first channel 30. The suction channel 50 and the instrument insertion channel 46 merge/join in a merging portion of the third joined channel 38. The merging portion may extend towards the proximal working channel tube portion 52.

The suction channel 50 comprises a suction channel central axis 54 extending along the suction channel 50. The instrument insertion channel 46 comprises an instrument insertion channel central axis or line 56 extending along the instrument insertion channel 46. The instrument insertion channel central axis or line 56 has a curvature corresponding to the curvature of the instrument insertion channel 46. It is preferred that the instrument insertion channel 46 is configured to pre-bend any instrument inserted into the instrument insertion channel 46 due to the curvature thereof. The first opening 28 may be in a plane which is tilted somewhat relative to the instrument insertion channel central axis or line 56, i.e. not at right angles thereto. A first part of the instrument insertion channel 46 may further have a different curvature than the rest of the instrument insertion channel 46. Additionally or alternatively any biopsy cap attached to the biopsy port may further add to the complexity of the instrument routing. The instrument insertion channel 46 may be configured to avoid any direct line of sight from the first opening 28 to the first portion 44 of the joined channel 38. The third joined channel 38 comprises a central axis 58. The instrument insertion channel central axis or line 56 and the suction channel central axis 54 are both offset/ displaced with respect to the central axis 58 of the third joined channel 38 due to the provision of the first portion 44 of the third joined channel 38 forming an extension of the first channel 30 and of the second portion 48 of the third joined channel 38 forming an extension of the second channel 36. The proximal working channel tube portion 52 comprises a proximal working channel tube portion central axis 60. The proximal working channel tube portion central axis 60 is parallel to the suction channel central axis 54.

Figures 3 and 4 both show perspective views of the Y-connector 22 according to the present disclosure. The first channel 30 inside the first branch or socket 26 and the first portion 44 of the third joined channel 38 inside the third branch or socket 40 form together the instrument insertion channel 46. The second channel 36 inside the second branch or socket 32 and a second portion 48 of the third joined channel 38 form together the suction channel 50. The suction channel 50 and the instrument insertion channel 46 are offset or displaced from/ with respect to each other. I.e. the suction channel central axis 54 is offset from the central axis 58 of the third joined channel 38. As shown in Fig. 4, the curved instrument insertion central axis or line 56 lies in a first plane 62. More concretely, the curved instrument insertion central axis 56 defines the first plane 62 and lies completely in the first plane 62. The suction channel central axis 54 does not lie in the first plane 62 and intersects with the first plane 62 in an intersecting point or extends parallel to the first plane 62. The Y-connector 22 comprises first fastening means 64 extending from the outer side of the first branch or socket 26 and the second branch or socket 32. Second fastening means 66 are formed as towers extending from the first branch or socket 26 toward the outside. The fastening means 64, 66 are configured and provided to connect the Y-connector 22 to the endoscope handle 4 by a press-fit connection and configured to allow for some tolerance in dimension of the parts by the oblong shape.

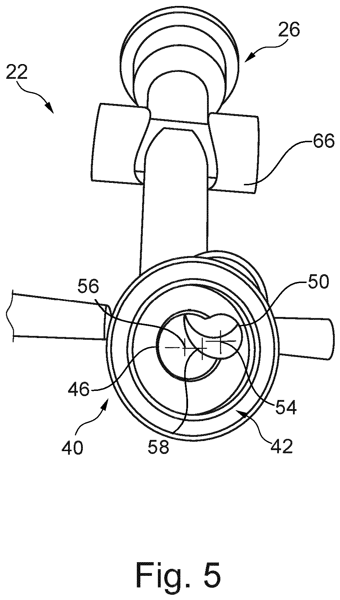

Fig. 5 shows another perspective view of the Y-connector 22. The instrument insertion channel 46 and the suction channel 50 extend adjacent to each other and merge inside the third branch or socket 40. The suction channel central axis 54 and the instrument insertion channel central axis or line 56 are offset or displaced to the central axis 58 of the third joined channel 38 in the cross-section of the third opening 42 and also inside the third branch or socket 40. Thus, the instrument insertion channel 46 and the suction channel 50 form a cross-section in the shape of connected circles inside the third branch or socket 40. Looking from the front side of the Y-connector 22, the third opening 42 has the shape of two connected or intersecting circles. The instrument insertion channel 46 and the suction channel 50 are both offset with respect to the central axis 58 of the third joined channel 38.

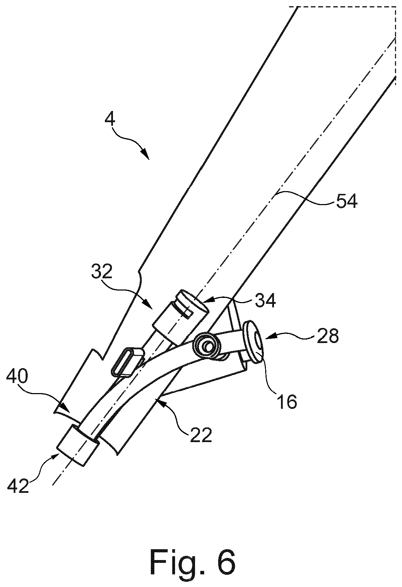

Fig. 6 shows a perspective view of the Y-connector 22 being inserted into a portion of the endoscope handle or interface 4. The suction channel central axis 54 extends parallel or along a main extension direction or a longitudinal axis or extension of the endoscope handle or interface 4. The working channel access port 16 extends to the outside of the endoscope handle or interface 4. Through the working channel access port 16 and the first opening 28, a surgical instrument 25 is inserted into the Y-connector 22 and thus into the working channel 14. The third opening 42 is connected to the working channel tube 18 (not shown in Fig. 6). A suction pipe (not shown in Fig. 6) is connected to the second opening 34 to suck liquid or mucus through the suction channel 50 of the Y-connector 22 out of the working channel tube 18.

### List of reference signs

- 2: endoscope
- 4: endoscope handle
- 6: insertion cord
- 8: insertion tube
- 10: bending section
- 12: distal tip unit
- 14: working channel
- 16: working channel access port
- 18: working channel tube
- 20: operating unit
- 22: Y-connector
- 24: connecting cable
- 24a: electrical connector
- 25: surgical instrument or tool
- 26: first branch or socket
- 28: first opening
- 30: first channel
- 32: second branch or socket
- 34: second opening
- 36: second channel
- 38: third joined channel
- 40: third branch or socket
- 42: third opening
- 44: first portion
- 46: instrument insertion channel
- 48: second portion
- 50: suction channel
- 51: suction tube
- 52: proximal working channel tube portion
- 53: radial inner wall surface
- 54: suction channel central axis
- 55: radial outer wall surface
- 56: instrument insertion channel central axis
- 58: central axis of third joined channel
- 60: proximal working channel tube portion central axis
- 62: first plane
- 64: first fastening means
- 66: second fastening means

## Claims

1. An endoscope (2) comprising:
an endoscope handle or interface (4); and
an insertion cord (6) extending from the endoscope handle or interface (4) and configured to be inserted into a patient's body cavity;
the endoscope handle or interface (4) comprising a Y-connector (22) having:
a first branch or socket (26) having a first opening (28) from which a first channel (30) extends;
a second branch or socket (32) having a second opening (34) from which a second channel (36) extends; and
a third branch or socket (40) having a third opening (42), wherein the first channel (30) and the second channel (36) join each other in a third joined channel (38) and the third joined channel (38) extends inside the third branch or socket towards the third opening; wherein
the first channel (30) and a first portion (44) of the third joined channel (38) form together an instrument insertion channel (46) of the Y-connector (22), the second channel (36) and a second portion (48) of the third joined channel (38) form together a suction channel (50) of the Y-connector (22), and a working channel tube (18) is directly or indirectly connected to the third joined channel (38) and extends from the endoscope handle or interface (4) into the insertion cord (6), wherein
the instrument insertion channel (46) and the suction channel (50) are at least in portions displaced or offset with respect to each other, wherein
the instrument insertion channel (46) is a curved channel and has a curved instrument insertion channel central axis or line (56), wherein the curved instrument insertion channel central axis or line (56) defines a first plane (62), wherein
the curved instrument insertion channel central axis or line (56) lies completely in the first plane (62), **characterized in that**
the suction channel (50) has a suction channel central axis (54) that does not lie in the first plane (62).

2. The endoscope (2) according to claim 1, wherein the suction channel central axis (54) is parallel to the first plane (62) or crosses the first plane (62) at an intersection point.

3. The endoscope (2) according to claim 1 or 2, wherein:
the suction channel (50) is a straight channel; wherein
a proximal working channel tube portion (52) of the working channel tube (18) is directly or indirectly connected to the third joined channel (38), the proximal working channel tube portion (52) comprising a proximal working channel tube portion central axis (60); and wherein
the suction channel central axis (54) is parallel to the proximal working channel tube portion central axis (60) and the suction channel (50) forms a continuation of the proximal working channel tube portion (52).

4. The endoscope (2) according to claim 3, wherein the suction channel central axis (54) corresponds to the proximal working channel tube portion central axis (60).

5. The endoscope (2) according to any one of claims 1 to 4, wherein a cross-sectional area of the suction channel (50) increases in the second channel (36) towards the second opening (34).

6. The endoscope (2) according to any one of claims 1 to 5, wherein the first channel (30) is a curved channel having a curvature at least in sections inside the first branch or socket (26) and/or the first portion (44) of the third joined channel (38) is or forms a curved channel having a curvature at least in sections inside the third branch or socket (40).

7. The endoscope (2) according to any one of the preceding claims 1 to 6, wherein the instrument insertion channel (46) and the suction channel (50) merge inside the third branch or socket (40) and form a cross-section in the shape of connected circles inside the third branch or socket (40).

8. The endoscope (2) according to any one of the preceding claims 1 to 7, wherein the third opening (42) has a shape of connected circles.

9. The endoscope (2) according to any one of the preceding claims 1 to 8, wherein a suction channel central axis (54) and an instrument insertion channel central axis or line (56) are displaced or offset with respect to a center point or central axis (58) of the third opening (42).

10. The endoscope (2) according to any one of the preceding claims 1 to 9, wherein a diameter or circumference of the suction channel (50) is smaller than a diameter or circumference of the instrument insertion channel (46).

11. The endoscope (2) according to any one of the preceding claims 1 to 10, wherein the Y-connector (22) is manufactured as an integral piece, preferably from a plastic material, especially preferred by injection molding.

12. The endoscope (2) according to any one of the preceding claims 1 to 11, wherein the first channel (30) is formed to be configured to pre-bend an instrument inserted into the instrument insertion channel (46) inside the first branch or socket (26) through its curvature.

13. Visualization system comprising: an endoscope (2) according to any one of claims 1 to 12; and a monitor (M) connectable to the endoscope (2).

## Patentansprüche

1. Endoskop (2) aufweisend:
einen Endoskopgriff oder -schnittstelle (4); und
einen Einführstrang (6), der sich von dem Endoskopgriff oder -schnittstelle (4) erstreckt und so ausgebildet ist, dass er in die Körperhöhle eines Patienten eingeführt werden kann;
wobei der Endoskopgriff oder -schnittstelle (4) einen Y-Verbinder (22) aufweist, mit:
einem ersten Abzweig oder Anschluss (26) mit einer ersten Öffnung (28), von der sich ein erster Kanal (30) erstreckt;
einem zweiten Abzweig oder Anschluss (32) mit einer zweiten Öffnung (34), von der sich ein zweiter Kanal (36) erstreckt; und
einem dritten Abzweig oder Anschluss (40) mit einer dritten Öffnung (42), wobei der erste Kanal (30) und der zweite Kanal (36) in einem dritten Verbindungskanal (38) zusammenlaufen und sich der dritte Verbindungskanal (38) innerhalb des dritten Abzweigs oder Anschlusses in Richtung der dritten Öffnung erstreckt; wobei
der erste Kanal (30) und ein erster Abschnitt (44) des dritten Verbindungskanals (38) zusammen einen Instrumenteneinführkanal (46) des Y-Verbinders (22) bilden, der zweite Kanal (36) und ein zweiter Abschnitt (48) des dritten Verbindungskanals (38) zusammen einen Saugkanal (50) des Y-Verbinders (22) bilden, und ein Arbeitskanalschlauch (18) direkt oder indirekt mit dem dritten Verbindungskanal (38) verbunden ist und sich von dem Endoskopgriff oder -schnittstelle (4) in den Einführstrang (6) erstreckt, wobei
der Instrumenteneinführkanal (46) und der Saugkanal (50) zumindest abschnittsweise zueinander versetzt oder verschoben sind, wobei
der Instrumenteneinführkanal (46) ein gekrümmter Kanal ist und eine gekrümmte Instrumenteneinführkanal-Mittelachse oder -linie (56) aufweist, wobei die gekrümmte Instrumenteneinführkanal-Mittelachse oder -linie (56) eine erste Ebene (62) definiert, wobei
die Mittelachse oder -linie (56) des gekrümmten Instrumenteneinführkanals vollständig in der ersten Ebene (62) liegt, **dadurch gekennzeichnet, dass**
der Saugkanal (50) eine Saugkanal-Mittelachse (54) aufweist, die nicht in der ersten Ebene (62) liegt.

2. Endoskop (2) nach Anspruch 1, wobei die Saugkanal-Mittelachse (54) parallel zu der ersten Ebene (62) verläuft oder die erste Ebene (62) in einem Schnittpunkt schneidet.

3. Endoskop (2) nach Anspruch 1 oder 2, wobei:
der Saugkanal (50) ein gerader Kanal ist; wobei
ein proximaler Arbeitskanalschlauchabschnitt (52) des Arbeitskanalschlauchs (18) direkt oder indirekt mit dem dritten Verbindungskanal (38) verbunden ist, wobei der proximale Arbeitskanalschlauchabschnitt (52) eine proximale Arbeitskanalschlauchabschnitt-Mittelachse (60) aufweist; und wobei
die Saugkanal-Mittelachse (54) parallel zu der proximalen Arbeitskanalschlauchabschnitt-Mittelachse (60) verläuft und der Saugkanal (50) eine Fortsetzung des proximalen Arbeitskanalschlauchabschnitts (52) bildet.

4. Endoskop (2) nach Anspruch 3, wobei die Saugkanal-Mittelachse (54) mit der proximalen Arbeitskanalschlauchabschnitt-Mittelachse (60) übereinstimmt.

5. Endoskop (2) nach einem der Ansprüche 1 bis 4, wobei sich die Querschnittsfläche des Saugkanals (50) in dem zweiten Kanal (36) in Richtung der zweiten Öffnung (34) vergrößert.

6. Endoskop (2) nach einem der Ansprüche 1 bis 5, wobei der erste Kanal (30) ein gekrümmter Kanal ist, der zumindest abschnittsweise innerhalb des ersten Abzweigs oder Anschlusses (26) eine Krümmung aufweist, und/oder der erste Abschnitt (44) des dritten Verbindungskanals (38) ein gekrümmter Kanal ist oder einen gekrümmten Kanal bildet, der zumindest abschnittsweise innerhalb des dritten Abzweigs oder Anschlusses (40) eine Krümmung aufweist.

7. Endoskop (2) nach einem der vorhergehenden Ansprüche 1 bis 6, wobei der Instrumenteneinführkanal (46) und der Saugkanal (50) innerhalb des dritten Abzweigs oder Anschlusses (40) zusammenlaufen und innerhalb des dritten Abzweigs oder Anschlusses (40) einen Querschnitt in Form miteinander verbundener Kreise bilden.

8. Endoskop (2) nach einem der vorhergehenden Ansprüche 1 bis 7, wobei die dritte Öffnung (42) die Form miteinander verbundener Kreise aufweist.

9. Endoskop (2) nach einem der vorhergehenden Ansprüche 1 bis 8, wobei die Saugkanal-Mittelachse (54) und die Instrumenteneinführkanal-Mittelachse oder -linie (56) gegenüber einem Mittelpunkt oder einer Mittelachse (58) der dritten Öffnung (42) versetzt oder verschoben sind.

10. Endoskop (2) nach einem der vorhergehenden Ansprüche 1 bis 9, wobei der Durchmesser oder Umfang des Saugkanals (50) kleiner ist als der Durchmesser oder Umfang des Instrumenteneinführkanals (46).

11. Endoskop (2) nach einem der vorhergehenden Ansprüche 1 bis 10, wobei der Y-Verbinder (22) als einteiliges Bauteil, vorzugsweise aus Kunststoff, besonders bevorzugt im Spritzgussverfahren, hergestellt ist.

12. Endoskop (2) nach einem der vorhergehenden Ansprüche 1 bis 11, wobei der erste Kanal (30) so ausgebildet ist, dass er ein in den Instrumenteneinführkanal (46) innerhalb des ersten Abzweigs oder Anschlusses (26) eingeführtes Instrument durch seine Krümmung vorbiegt.

13. Bildgebungssystem, aufweisend: ein Endoskop (2) nach einem der Ansprüche 1 bis 12, und einen Monitor (M), der mit dem Endoskop (2) verbunden werden kann.

## Revendications

1. Endoscope (2) comprenant :
une poignée ou une interface d'endoscope (4) ; et
un cordon d'insertion (6) s'étendant de la poignée ou de l'interface d'endoscope (4) et configuré pour être inséré dans une cavité corporelle d'un patient ;
la poignée ou l'interface d'endoscope (4) comprenant un connecteur en Y (22) présentant :
une première branche ou douille (26) présentant une première ouverture (28) à partir de laquelle s'étend un premier canal (30) ;
une deuxième branche ou douille (32) présentant une seconde ouverture (34) à partir de laquelle s'étend un deuxième canal (36) ; et
une troisième branche ou douille (40) présentant une troisième ouverture (42), dans lequel le premier canal (30) et le deuxième canal (36) se rejoignent en un troisième canal joint (38) et le troisième canal joint (38) s'étend à l'intérieur de la troisième branche ou douille vers la troisième ouverture ; dans lequel
le premier canal (30) et une première partie (44) du troisième canal joint (38) forment ensemble un canal d'insertion d'instrument (46) du connecteur en Y (22), le deuxième canal (36) et une deuxième partie (48) du troisième canal joint (38) forment ensemble un canal d'aspiration (50) du connecteur en Y (22), et un tube de canal de travail (18) est connecté directement ou indirectement au troisième canal joint (38) et s'étend de la poignée ou de l'interface d'endoscope (4) jusqu'au cordon d'insertion (6), dans lequel
le canal d'insertion d'instrument (46) et le canal d'aspiration (50) sont au moins partiellement déplacés ou décalés l'un par rapport à l'autre, dans lequel
le canal d'insertion d'instrument (46) est un canal courbe et présente un axe ou une ligne centrale de canal d'insertion d'instrument courbe (56), dans lequel l'axe ou la ligne centrale de canal d'insertion d'instrument courbe (56) définit un premier plan (62), dans lequel
l'axe ou la ligne centrale de canal d'insertion de l'instrument courbe (56) se situe entièrement dans le premier plan (62), **caractérisé en ce que**
le canal d'aspiration (50) présente un axe central de canal d'aspiration (54) qui ne se trouve pas dans le premier plan (62).

2. Endoscope (2) selon la revendication 1, dans lequel l'axe central de canal d'aspiration (54) est parallèle au premier plan (62) ou traverse le premier plan (62) en un point d'intersection.

3. Endoscope (2) selon la revendication 1 ou 2, dans lequel :
le canal d'aspiration (50) est un canal droit ; dans lequel
une partie proximale de tube de canal de travail (52) du tube de canal de travail (18) est connectée directement ou indirectement au troisième canal joint (38), la partie proximale de tube de canal de travail (52) comprenant un axe central de partie proximale de tube de canal de travail (60) ; et dans lequel
l'axe central de canal d'aspiration (54) est parallèle à l'axe central de partie proximale de tube de canal de travail (60) et le canal d'aspiration (50) forme une continuation de la partie proximale de tube de canal de travail (52).

4. Endoscope (2) selon la revendication 3, dans lequel l'axe central de canal d'aspiration (54) correspond à l'axe central de partie proximale de tube de canal de travail (60).

5. Endoscope (2) selon l'une quelconque des revendications 1 à 4, dans lequel une section transversale du canal d'aspiration (50) augmente dans le deuxième canal (36) vers la deuxième ouverture (34).

6. Endoscope (2) selon l'une quelconque des revendications 1 à 5, dans lequel le premier canal (30) est un canal courbe présentant une courbure au moins dans certaines sections à l'intérieur de la première branche ou douille (26) et/ou la première partie (44) du troisième canal joint (38) est ou forme un canal courbe présentant une courbure au moins dans les sections à l'intérieur de la troisième branche ou douille (40).

7. Endoscope (2) selon l'une quelconque des revendications précédentes 1 à 6, dans lequel le canal d'insertion d'instrument (46) et le canal d'aspiration (50) fusionnent à l'intérieur de la troisième branche ou douille (40) et forment une section transversale en forme de cercles connectés à l'intérieur de la troisième branche ou douille (40).

8. Endoscope (2) selon l'une quelconque des revendications précédentes 1 à 7, dans lequel la troisième ouverture (42) présente une forme de cercles connectés.

9. Endoscope (2) selon l'une quelconque des revendications précédentes 1 à 8, dans lequel un axe central de canal d'aspiration (54) et un axe ou une ligne centrale de canal d'insertion d'instrument (56) sont déplacés ou décalés par rapport à un point central ou axe central (58) de la troisième ouverture (42).

10. Endoscope (2) selon l'une quelconque des revendications précédentes 1 à 9, dans lequel un diamètre ou une circonférence du canal d'aspiration (50) est inférieur à un diamètre ou une circonférence du canal d'insertion d'instrument (46).

11. Endoscope (2) selon l'une quelconque des revendications précédentes 1 à 10, dans lequel le connecteur en Y (22) est fabriqué comme une pièce intégrale, de préférence en matière plastique, de manière particulièrement préférée par moulage par injection.

12. Endoscope (2) selon l'une quelconque des revendications précédentes 1 à 11, dans lequel le premier canal (30) est formé pour être configuré pour pré-courber un instrument inséré dans le canal d'insertion d'instrument (46) à l'intérieur de la première branche ou douille (26) par le biais de sa courbure.

13. Système de visualisation comprenant : un endoscope (2) selon l'une quelconque des revendications 1 à 12 ; et un moniteur (M) pouvant être connecté à l'endoscope (2).
